# FASCICULE DE BREVET EUROPEEN

(11) **EP 1 981 443 B1**
(45) Date de publication et mention de la délivrance du brevet: **15.06.2011**
(21) Numéro de dépôt: 06847072.3
(22) Date de dépôt: 19.12.2006
(51) Int. Cl.: A61F 2/44

(54) **ESPACEUR DISCAL ANATOMIQUE**
ANATOMISCHER INTERVERTEBRAL-ABSTANDSHALTER
ANATOMIC INTERVERTEBRAL SPACER

(30) Priorité: 20.12.2005 FR 0512979
(43) Date de publication de la demande: 22.10.2008
(73) Titulaire: Spineart SA, 1217 Meyrin (CH)
(72) Inventeur: LEVIEUX, Jérôme, CH-1293 Bellevue (CH); TROPIANO, Patrick, F-13008 Marseille (FR)
(74) Mandataire: Perin, Georges
(86) Numéro de dépôt international: PCT/FR2006/002792
(87) Numéro de publication internationale: WO 2007/077319

(56) Documents cités:
- EP-A- 1 287 794
- EP-A- 1 391 188
- DE-A1- 10 253 169
- US-A- 5 545 229
- US-A- 5 702 450
- US-A1- 2001 016 773
- US-A1- 2004 220 672
- US-A1- 2005 165 485

## Description

La présente invention concerne un espaceur discal anatomique et ses applications.

Les pathologies rachidiennes lombaires sont actuellement traitées de différentes façons.

Les arthrodèses consistent à bloquer un disque afin de supprimer le mouvement entre deux vertèbres adjacentes. Elles s'opèrent par l'intermédiaire de cages introduites entre deux corps vertébraux. La cage est une sorte de boîte solide dont le centre est rempli d'os spongieux. Les parois des cages sont ajourées pour permettre la fusion avec les corps vertébraux. De telles cages sont par exemple décrites dans FR-A-2 851 457, 2 736 538, 2 703 580, 2 816 201, 2 808 673 et 2 790 945. Mais elles limitent les amplitudes de mouvement du patient. De plus, elles augmentent le travail des disques voisins, ce qui contribue à les user plus vite.

Les arthroplasties lombaires par prothèse de disque par voie antérieure représentent une alternative remédiant aux inconvénients de l'arthrodèse. On utilise ici des prothèses disposées à la place du disque et constituées de deux plateaux en contact avec les corps vertébraux entre lesquels est placée une pièce intermédiaire qui est soit une sphère soit une pièce en matériau élastique. Dans le cas du système avec sphère (dit ball-socket) le plateau supérieur possède dans sa face interne une forme sphérique congruante avec la sphère de la pièce intermédiaire de façon à obtenir les mouvement naturels de la colonne vertébrale. Dans le cas de pièce intermédiaire en matériau élastique c'est par déformation que les mouvements sont reproduits.

Ces techniques récemment utilisées présentent l'avantage de restituer les mouvements du disque. Mais leur mise en place peut-être délicate voire dangereuse. En effet la mise en place de prothèses par voie antérieure demande la mobilisation de l'aorte et le la veine cave, ce qui peut-être périlleux. Par ailleurs de nombreux chirurgiens du rachis ne sont pas habitués aux techniques par voie antérieure et doivent se faire aider par un chirurgien d'une autre spécialité (vasculaire par ex), ce qui représente évidemment un coût important et une mobilisation inutile de compétences.

FR-A-2 858 546 décrit des prothèses réalisées en deux parties que l'ont peut implanter par voie postérieure. Mais les deux parties de la prothèse possèdent un tronc de sphère dont les centres doivent être confondus pour que la prothèse puisse fonctionner normalement. Ceci présente donc des difficultés de précision lors de la mise en place.

De plus dans le cas où une des deux parties bouge même légèrement lors de la vie du patient, les deux troncs de sphères n'ont plus de centre identique et l'ensemble ne peut donc plus fonctionner.

En outre ce type de prothèse ne peut pas être installé par accès postéro-latéral (type TLIF).

De surcroît, il ne permet pas l'amortissement des chocs

AU-A-4238202 décrit également une prothèse destinée à être implantée par voie postérieure. Mais sa forme cylindrique ne correspond pas aux habitudes des chirurgiens. En outre sa stabilité est douteuse. En effet, sans arthrodèse, les mouvements durent toute la vie du patient, et on peut donc évidemment craindre qu'un dévissage se produise dans le temps.

De plus une telle prothèse ne peut pas être implantée par voie postéro-tatérale. En outre elle est difficile à impacter dans les disques pincés.

L'art antérieur est représenté, par example, par US-A-5,545,229.

Il serait donc souhaitable de disposer d'un espaceur discal qui notamment puisse se déformer selon la demande des mouvements anatomiques, n'impose pas une cinématique particulière et qui puisse être installé par abord postéro-latéral.

Or après de longues recherches la demanderesse a mis au point une prothèse en une partie pouvant être installée par postéro-latéral remédiant à ces inconvénients.

L'invention se présente sous son aspect le plus général, sous la forme d'un espaceur discal multi couches comprenant deux éléments destinés à s'ancrer efficacement sur les plateaux vertébraux. Rappelons ici que les dispositifs de l'invention s'adressent au domaine des espaceurs postérieurs et non par exemple au domaine des espaceurs antérieurs. Il en résulte que les espaceurs antérieurs comprennent un seul élément et ont généralement une largeur de 25 à 40 mm, une épaisseur de 7 à 14 mm et une longueur de 20 à 30 mm, alors que les espaceurs pouvant être installés par abord postérieur ou postéro-latéral comme ceux comprenant deux éléments, étant donné qu'ils sont séparés par la dure mère, ont généralement une largeur de 8 à 12 mm, une épaisseur de 7 à 14 mm et une longueur de 20 à 26 mm. Les espaceurs en forme de haricot de l'invention, qui ne sont pas utilisés par paire, ont une largeur de 10 mm environ, une épaisseur de 7 à 14 mm et une longueur curviligne de 25 à 40 mm environ.

La présente demande a plus précisément pour objet un espaceur discal multi couches, caractérisé en ce qu'il comprend au moins trois couches superposées dont une couche inférieure, une couche supérieure, et au moins une couche intermédiaire, la au moins une couche intermédiaire étant réalisée en matériau différent de celui des couches inférieure et supérieure et moins rigide et en ce qu'il a les dimensions mentionnées au-dessus adaptées pour être installé par abord postéro-latéral.

Plus précisément encore, la présente demande a pour objet un espaceur discal multi couches, comprenant au moins trois couches superposées dont une couche inférieure, une couche supérieure, et au moins une couche intermédiaire, la au moins une couche intermédiaire étant réalisée en matériau différent de celui des couches inférieure et supérieure et moins rigide caractérisé en ce qu'il a, vu de dessus, une forme générale de graine de haricot.

Un espaceur discal est par nature destiné à être inséré entre deux vertèbres. Par convention on appellera donc couche "supérieure" la couche située du côté de la tête d'un individu debout appareillé. Les autres termes à signification directionnelle tels que "avant" et "arrière" ou "haut" et " bas" se réfèrent aussi à l'orientation de la cage lorsqu'elle est implantée dans la colonne vertébrale à appareiller.

Dans des conditions préférentielles de mise en oeuvre de l'invention, un espaceur discal multi couches ci-dessus comprend une seule couche intermédiaire.

La au moins une couche intermédiaire peut par exemple être réalisée en matériau élastique, de préférence en matière plastique élastomère, particulièrement en matière plastique silicone poly addition ou polycondensation ou en matière plastique polyuréthane et tout particulièrement en polyuréthane élastomère tel que celui commercialisé par la société POLY MEDICA INDUSTRIES, Inc (Massachusetts) sous la dénomination Chronoflex®. Une couche intermédiaire peut être réalisée en un matériau unique ou un mélange des deux matériaux comme un mélange de matière plastique silicone et de polyuréthane.

Dans d'autres conditions préférentielles de mise en oeuvre de l'invention, la au moins une couche intermédiaire comporte en outre une ou plusieurs lumières réparties régulièrement ou non dans ladite couche intermédiaire. On a ainsi la possibilité d'avoir différents types d'amortissement adaptés à la réponse recherchée.

D'une épaisseur appropriée, la au moins une couche intermédiaire est capable de reproduire les mouvements de l'articulation. Par exemple, si un homme se plie vers l'avant, le disque situé entre L4 et L5 devra s'écraser d'environ 2 mm en avant ; s'il se plie vers son côté droit, ce disque devra s'écraser dans sa partie droite d'environ 1,5mm. La pièce élastique aura donc la hauteur nécessaire pour permettre les amplitudes de préférence maximales demandées par les mouvements anatomiques. La pièce élastique pourra aussi avoir la hauteur nécessaire pour permettre au moins 50%, avantageusement au moins 70%, notamment au moins 80%, tout particulièrement au moins 90% des amplitudes maximales demandées par les mouvements anatomiques.

La au moins une couche intermédiaire peut avoir une épaisseur de 2 à 12 mm, de préférence de 3 à 11 mm, notamment de 4 à 10 mm, tout particulièrement de 5 à 9 mm.

Dans la présente demande et dans ce qui suit, la rigidité du ou des matériaux de la au moins une couche intermédiaire est exprimée par leur dureté.

La dureté du ou des matériaux de la au moins une couche intermédiaire peut par exemple être comprise entre 40 Shore A et 60 Shore D, de préférence comprise entre 50 Shore A et 55 Shore D, notamment comprise entre 65 Shore A et 50 Shore D, particulièrement comprise entre 25 SHD et 45 Shore D, tout particulièrement comprise entre 30 Shore D et 40 Shore D.

On peut combiner au choix de l'épaisseur le choix de la dureté du ou des matériaux de la au moins une couche intermédiaire et le choix de la forme et de la taille de la ou des lumières pour obtenir l'effet d'amortissement désiré.

La au moins une couche intermédiaire peut être dédoublée dans son épaisseur et recevoir un insert, notamment en un autre matériau élastique que celui dont est constituée ladite au moins une couche intermédiaire. Cet insert peut éventuellement déboucher sur l'extérieur et constituer alors une couche intermédiaire supplémentaire. Le matériau élastique de l'insert sera habituellement plus souple (ou au contraire plus rigide) que celui dont est constituée ladite au moins une couche intermédiaire.

La couche inférieure et la couche supérieure forment des plaques qui sont réalisées en matériau dur et peuvent de préférence être réalisées en titane et avantageusement munies en surface d'un revêtement notamment de titane poreux seul ou accompagné d'hydroxyapatite. Classiquement, leur surface externe est de préférence crantée ou dentelée pour conférer à un espaceur discal selon l'invention une stabilité primaire et à long terme.

Dans encore d'autres conditions préférentielles de mise en oeuvre de l'invention, le côté de l'espaceur discal multi couches à insérer en premier entre deux vertèbres a en coupe verticale une forme effilée ou fuselée, par exemple en cône ou en obus.

Dans toujours d'autres conditions préférentielles de mise en oeuvre de l'invention, l'espaceur discal multi couches ci-dessus a une forme générale proche d'un parallélépipède rectangle, et dans ce cas s'utilise par paire, ou vu de dessus, une forme de graine de haricot.

Un espaceur discal multi couches objet de la présente invention peut notamment être réalisé par surmoulage entre les plaques formant la couche inférieure et la couche supérieure intérieurement munies d'aspérités.

Un espaceur discal multi couches objet de la présente invention possède de très intéressantes propriétés et qualités.

Grâce à sa couche intermédiaire élastique, il tolère les imprécisions de positionnement et se déforme selon la demande des mouvements anatomiques.

Une prothèse dans laquelle il y a un contact entre deux surfaces sphériques va générer des mouvements de rotation pure. Ce système impose donc la cinématique de la liaison rotulienne. Or, lors des mouvements naturels, les disques ont des mouvements aléatoires combinant rotation et translation. La présence d'un matériau élastique dans les espaceurs discaux de la présente invention permet aux espaceurs de se déformer comme le demande le corps sans imposer de trajectoire fixe. Les espaceurs discaux de la présente invention n'imposent pas une cinématique particulière.

En outre, ils permettent l'amortissement des chocs.

L'invention sera mieux comprise si l'on se réfère aux dessins annexés sur lesquels
- les figures 1, 2, 3 et 4 représentent des vues en perspective des espaceurs discales différents ;
- la figure 5 est une vue de dessus d'un espaceur selon la présente invention en forme de haricot du type représenté à la figure 3 , dans sa position d'installation définitive entre deux vertèbres;
- la figure 6 représente vus en coupe verticale frontale deux espaceurs du type représenté aux figures 1, 2 et 4, également installés entre deux vertèbres, et
- les figures 7 à 16 représentent, en coupe verticale, des espaceurs selon la présente invention munis de différents types de lumière.

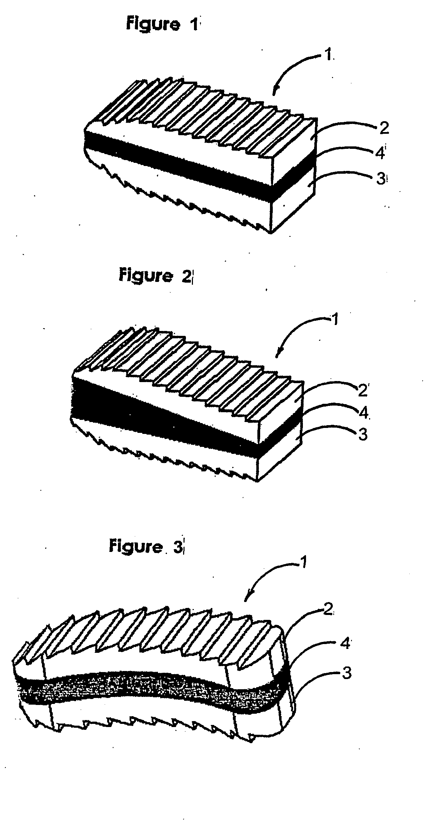

Sur la figure 1, on observe que l'espaceur discal 1 représenté comprend trois couches à savoir une couche supérieure 2 constituée d'une plaque de titane à la surface crantée, une couche inférieure 3, de même structure, et une couche intermédiaire 4 constituée, sur le modèle représenté, de polyuréthane de dureté 35 Shore D (polyuréthane élastomère commercialisé par la société POLY MEDICA INDUSTRIES, Inc (Massachusetts) sous la dénomination Chronoflex®).

On peut observer que la forme générale de l'espaceur discal 1 est celle d'un parallélépipède rectangle, ce qui ne forme pas partie de l'invention, dont toutefois une des extrémités, figurée à gauche sur le schéma, est fuselée. Une telle forme permet de l'insérer plus facilement entre deux vertèbres pincées.

Sur le modèle ici représenté, l'épaisseur de la couche intermédiaire 4 est constante sur toute la structure de l'espaceur discal.

Les plaques en titane supérieure 2 et inférieure 3 s'amincissent progressivement, pour obtenir le fuselage désiré.

Sur la figure 2, on peut observer sensiblement les mêmes caractéristiques que sur la figure 1, avec toutefois une couche intermédiaire 4 qui n'est pas d'épaisseur constante, mais qui va en s'épaississant, au fur et à mesure_que l'on approche de l'extrémité fuselée.

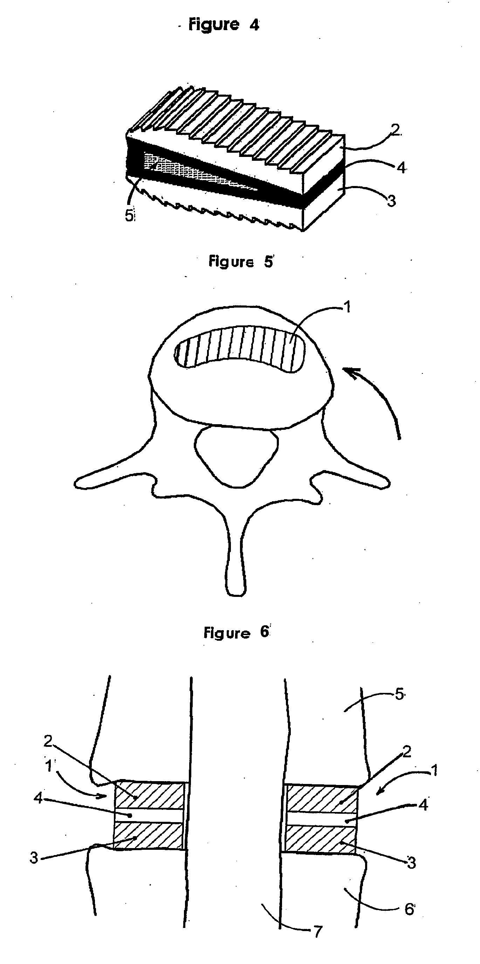

Les espaceurs représentés aux figures 1 et 2 s'utilisent par paire ce qui ne forme pas partie de l'invention, comme on le verra ci-après sur la figure 6.

Le modèle selon l'invention représenté à la figure 3, par contre, s'utilise seul, comme représenté à la figure 5. Sur l'espaceur discal représenté sur cette figure 3, on observe sensiblement les mêmes éléments que sur la figure 1, mais toutefois sa forme générale n'est pas celle d'un parallélépipède rectangle, mais, vue de dessus, une forme de graine de haricot.

Le modèle représenté à la figure 4 est une variante de celui représenté à la figure 2. Mais la couche intermédiaire 4 est composée de deux matières différentes et séparées. Un insert 5 est en effet réalisé dans un matériau différent de celui qui constitue la couche intermédiaire 4 au contact des plaques de titane 2 et 3. Dans une première version, l'insert 5 a été réalisé avec une matière plastique plus rigide. Dans une autre variante réalisée, l'insert est au contraire réalisé en un matériau moins rigide. Et dans une troisième variante, l'insert est un insert en titane.

Dans le modèle représenté, l'insert 5 est directement accessible sur les côtés, mais ne débouche pas à l'extérieur au niveau de la partie de l'espaceur discal 1 destinée à s'introduire en premier entre deux vertèbres, à gauche sur la figure.

Sur la figure 5, on peut observer, installé en place, un espaceur discal 1 du type représenté à la figure 3. La flèche représente le sens d'introduction de l'espaceur discal 1 par le chirurgien entre deux vertèbres.

La figure 6 représente, vus en coupe verticale frontale, deux espaceurs du type représenté à la figure 1, installés entre deux vertèbres adjacentes 5, 6. Au centre du dessin est représentée la dure mère 7.

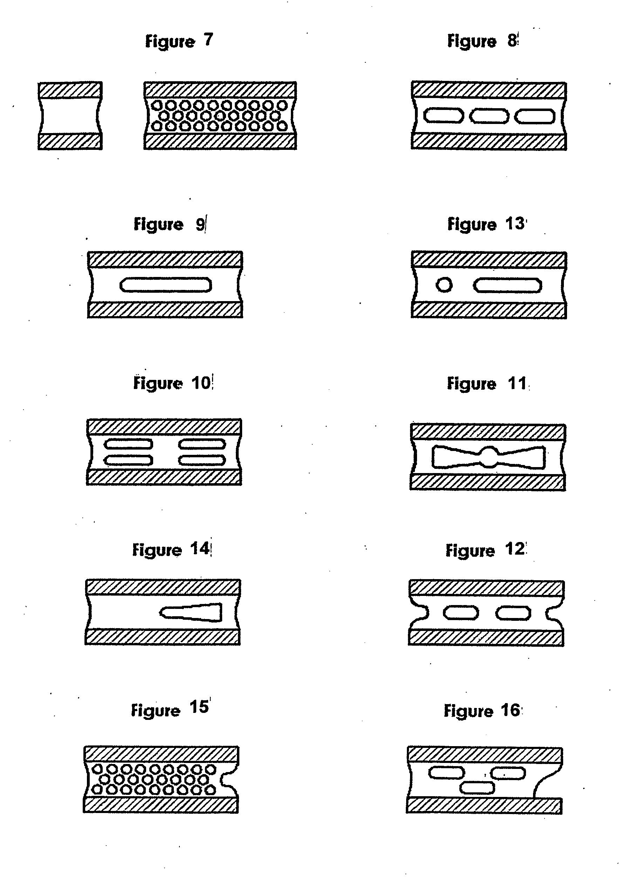

Les figures 7 à 16 représentent des espaceurs selon l'invention comprenant diverses lumières. Ces lumières sont multiples et réparties sensiblement symétriquement dans l'espaceur pour les espaceurs des figures 7 à 11.

Sur l'espaceur représenté à la figure 12, les extrémités longitudinales de l'espaceur sont sensiblement dépourvues de couche intermédiaire, en raison de la présence d'une lumière débouchant sur l'extérieur. Il en est de même pour les figures 15 et 16.

Avec les espaceurs représentés aux figures 7 et 8, on obtient une déformation homogène de l'espaceur.

Avec l'espaceur représenté à la figure 9 , la déformation au centre est plus importante ce qui augmente la capacité d'absorption des chocs.

Avec l'espaceur représenté à la figure 10, les deux lumières superposées augmentent la déformation à charge égale.

Avec l'espaceur représenté à la figure 11, les lumières importantes de chaque côté augmentent la capacité de déformation en flexion et en extension.

Avec l'espaceur représenté à la figure 12, les évidements de matière aux extrémités favorisent encore la capacité de déformation en flexion et en extension.

Avec l'espaceur représenté à la figure 13, les lumières sont disposées de manière asymétrique afin de favoriser la flexion ou l'extension.

Avec l'espaceur représenté à la figure 14, la lumière installée d'un seul côté favorise la flexion ou l'extension. Si la lumière est placée sur le côté postérieur on favorise l'extension, si la lumière est placée sur le côté antérieur on favorise la flexion.

Avec les espaceurs représentés aux figures 15 et 16, on combine déformation homogène et évidement d'un seul côté pour favoriser la flexion ou l'extension. Si l'évidement est placé du côté postérieur on favorise l'extension, s'il est placé du côté antérieur on favorise la flexion.

## Revendications

1. Un espaceur discal (1) multi couches, comprenant au moins trois couches superposées dont une couche inférieure (3), une couche supérieure (2), et au moins une couche intermédiaire (4), la au moins une couche intermédiaire (4) étant réalisée en matériau différent de celui des couches inférieure (3) et supérieure (2) et moins rigide, **caractérisé ce qu'**il a des dimensions adaptées pour être installé par abord postéro-latéral, en ce qu'il a, vu de dessus, une forme générale de graine de haricot et en ce qu'il a une largeur de 10 mm environ, une épaisseur de 7 à 14 mm et une longueur curviligne de 25 à 40 mm environ.

2. Un espaceur discal multi couches selon la revendication 1, **caractérisé en ce que** le côté de l'espaceur discal (1) multi couches à insérer en premier entre deux vertèbres a en coupe verticale une forme effilée ou fuselée.

3. Un espaceur discal multi couches selon la revendication 1 ou 2, **caractérisé en ce que** la au moins une couche Intermédiaire (4) comporte en outre une ou plusieurs lumières réparties régulièrement ou non dans ladite couche intermédiaire (4).

4. Un espaceur discal multi couches selon l'une des revendications 1 à 3, **caractérisé en ce qu'**il comprend une seule couche intermédiaire (4).

5. Un espaceur discal multi couches selon l'une des revendications 1 à 4, **caractérisé en ce que** la au moins une couche intermédiaire (4) est réalisée en matériau élastique, tel qu'en matière plastique élastomère, comme en matière plastique silicone poly addition ou polycondensation ou en matière plastique polyuréthane.

6. Un espaceur discal multi couches selon l'une des revendications 1 à 5. **caractérisé en ce que** la au moins une couche intermédiaire (4) a une épaisseur de 3 à 11 mm.

7. Un espaceur discal multi couches selon l'une des revendications 1 à 6, **caractérisé en ce que** la dureté du ou des matériaux de la au moins une couche intermédiaire (4) est comprise entre 50 Shore A et 55 Shore D.

8. Un espaceur discal multi couches selon l'une des revendications 1 à 7, **caractérisé en ce que** la au moins une couche intermédiaire (4) est dédoublée dans son épaisseur et reçoit un insert (5).

## Claims

1. A multilayer intervertebral spacer (1), comprising at least three superimposed layers including a lower layer (3), an upper layer (2), and at least one intermediate layer (4), the at least one intermediate layer (4) being made of a different and less rigid material than that of the lower (3) and upper (2) layers, **characterized in that** it has dimensions suitable for being installed by posterior or posterolateral access, **in that** viewed from above, it has the general shape of a kidney bean and **in that** it has a width of approximately 10 mm, a thickness of 7 to 14 mm and a curvilinear length of approximately 25 to 40 mm..

2. A multilayer intervertebral spacer according to claim 1, **characterized in that** the side of the multilayer intervertebral spacer (1) to be inserted first between two vertebrae has a tapered or spindle shape in vertical cross-section.

3. A multilayer intervertebral spacer according to claim 1 or 2, **characterized in that** the at least one intermediate layer (4) comprises moreover one or more lumina distributed regularly or irregularly in said intermediate layer (4).

4. A multilayer intervertebral spacer according to one of claims 1 to 3, **characterized in that** it comprises a single intermediate layer (4).

5. A multilayer intervertebral spacer according to one of claims 1 to 4, **characterized in that** the at least one intermediate layer (4) is made of elastic material, such as elastomer plastic material, such as polyaddition or polycondensation silicone plastic material or polyurethane plastic material.

6. A multilayer intervertebral spacer according to one of claims 1 to 5, **characterized in that** the at least one intermediate layer (4) has a thickness of 3 to 11 mm.

7. A multilayer intervertebral spacer according to one of claims 1 to 6, **characterized in that** the hardness of the material(s) of the at least one intermediate layer (4) is comprised between 50 Shore A and 55 Shore D.

8. A multilayer intervertebral spacer according to one of claims 1 to 7, **characterized in that** the at least one intermediate layer (4) is split in its thickness and receives an insert (5).

## Patentansprüche

1. Mehrschichtiger intervertebraler Abstandshalter (1) mit mindestens drei aufeinanderliegenden Schichten, nämlich einer unteren Schicht (3), einer oberen Schicht (2) und mindestens einer Zwischenschicht (4), wobei die mindestens eine Zwischenschicht (4) aus einem anderen Material als dasjenige der unteren (3) und oberen (2) Schichten und weniger steif ausgebildet ist, **dadurch gekennzeichnet, dass** seine Abmessungen geeignet sind, um durch einen hinteren, seitlichen Zugang eingesetzt zu werden, dass er, von oben gesehen, die allgemeine Form einer Bohne aufweist und dass er eine Breite von ca. 10 mm, eine Dicke von 7 bis 14 mm und eine gekrümmte Länge von ca. 25 bis 40 mm hat.

2. Mehrschichtiger intervertebraler Abstandshalter nach Anspruch 1, **dadurch gekennzeichnet, dass** die als erste zwischen zwei Wirbeln einzusetzende Seite des mehrschichtigen intervertebralen Abstandshalters (1) im Querschnitt eine spitz zulaufende oder sich verjüngende Form hat.

3. Mehrschichtiger intervertebraler Abstandshalter nach Anspruch 1 oder 2, **dadurch gekennzeichnet, dass** die mindestens eine Zwischenschicht (4) zudem einen Schlitz oder mehrere Schlitze aufweist, die in der Zwischenschicht (4) gleichmäßig oder nicht verteilt sind.

4. Mehrschichtiger intervertebraler Abstandshalter nach einem der Ansprüche 1 bis 3, **dadurch gekennzeichnet, dass** er eine einzige Zwischenschicht (4) aufweist.

5. Mehrschichtiger intervertebraler Abstandshalter nach einem der Ansprüche 1 bis 4, **dadurch gekennzeichnet, dass** die mindestens eine Zwischenschicht (4) aus einem elastischen Material wie aus ElastomerKunststoff, aus Polyadditions- oder Polykondensations-Silikon-Kunststoff besteht.

6. Mehrschichtiger intervertebraler Abstandshalter nach einem der Ansprüche 1 bis 4, **dadurch gekennzeichnet, dass** die mindestens eine Zwischenschicht (4) eine Dicke von 3 bis 11 mm aufweist.

7. Mehrschichtiger intervertebraler Abstandshalter nach einem der Ansprüche 1 bis 6, **dadurch gekennzeichnet, dass** die Härte des Materials oder der Materialen der mindestens einen Zwischenschicht (4) zwischen 50 Shore A und 55 Shore D beträgt.

8. Mehrschichtiger intervertebraler Abstandshalter nach einem der Ansprüche 1 bis 7, **dadurch gekennzeichnet, dass** die mindestens eine Zwischenschicht (4) in ihrer Dicke gespalten ist und eine Einlage (5) aufnimmt.
